(19) Europäisches Patentamt
European Patent Office
Office européen des brevets

(11) Numéro de publication : **0 560 684 A1**

(12) # DEMANDE DE BREVET EUROPEEN

(21) Numéro de dépôt : **93400626.3**

(22) Date de dépôt : **11.03.93**

(51) Int. Cl.$^5$ : **C07D 209/12,** A61K 7/13

(30) Priorité : **13.03.92 FR 9203050**

(43) Date de publication de la demande :
**15.09.93 Bulletin 93/37**

(84) Etats contractants désignés :
**AT BE CH DE DK ES FR GB GR IE IT LI NL PT SE**

(71) Demandeur : **L'OREAL**
**14, Rue Royale**
**F-75008 Paris (FR)**

(72) Inventeur : **Henrion, Jean-Christophe**
**8, rue Jolly**
**F-94160 Sain-Mandé (FR)**
Inventeur : **Philippe, Michel**
**3, rue de l'Aubépine**
**F-92160 Antony (FR)**
Inventeur : **Hocquaux, Michel**
**70, rue du Rendez-vous**
**F-75012 Paris (FR)**

(74) Mandataire : **Casalonga, Axel et al**
**BUREAU D.A. CASALONGA - JOSSE**
**Morassistrasse 8**
**D-80469 München (DE)**

(54) **Composés 1,4-naphtoquinone ou 1,4-naphtalènediol di- ou tri-indolylsubstitués, leurs procédés de préparation et leur utilisation en coloration des matières kératiniques.**

(57) Composés de formule :

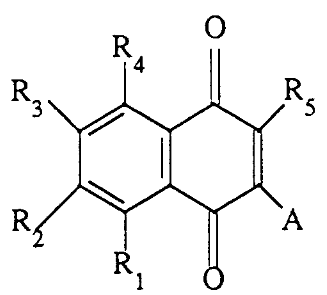

(I)

ou

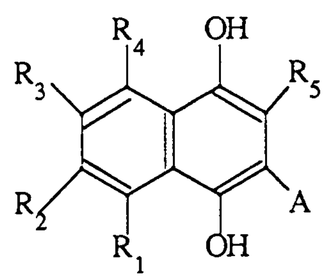

(II)

dans lesquelles :
$R_1$ désigne H, alkyle, OH ;
$R_4$ désigne H, alkyle, avec $R_1$, OH, C ;
$R_3$ désigne H, alkyle, halogène ;
$R_2$ et $R_5$ désignent H, halogène, alkyle, B ;
un seul pouvant désigner B ;
A, B, C, identiques ou différents, désignent un groupe de formule :

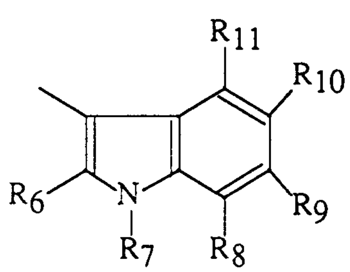

(III)

où

EP 0 560 684 A1

$R_6$ désigne H, alkyle, phényle ;
$R_7$ désigne H, alkyle ;
$R_8$ à $R_{11}$ , identiques ou différents, désignent H, alkyle, benzyloxy, halogène, alcoxy, OH, $NH_2$, éventuellement monosubstitué par un alkyle ou hydroxyalkyle avec deux au plus, désignent OH, alcoxy ou benzyloxy, un au plus désigne $NH_2$, éventuellement monosubstitué ;
sous réserve que :

— $R_1$ désigne OH si $R_4$ désigne C ;
— $R_4$ désigne C si $R_2$ désigne B ;
— $R_5$ désigne B si $R_4$ ne désigne pas C ;
— $R_4$ désigne C si $R_2$ et $R_6$ différents de B.

La présente invention concerne des composés 1,4-naphtoquinone ou 1,4-naphtalènediol di- ou tri-indolyl-substitués, leurs procédés de préparation ainsi que leur utilisation dans la coloration des matières kératiniques, en particulier des cheveux humains, de la peau ou des ongles ou dans des produits de maquillage.

On connaît dans l'état de la technique des produits de réaction d'addition de composés indoliques avec des quinones tels que la 1,4-naphtoquinone et ses dérivés. Les différentes structures plus ou moins complexes des produits résultants ont été étudiées dans les travaux de MOLHAU & REDLICH (Ber., 44, 3605; 1911), de BU'LOCK & MASON (J. Chem. Soc., pages 703 à 716, 1951), de PRASAD (Tetrahedron letters n° 15, pages 1361-1362, 1974) et de PROTA (Gazetta Chem. Ital.; 119, 153, 1989).

Les demandes EP-0376776 et 0460996 décrivent un procédé de teinture en deux étapes, mettant en oeuvre des mono- ou des dihydroxy indoles ou des aminoindoles qui sont oxydés par des quinones, pour former un pigment mélanique, in situ dans les fibres kératiniques.

La demanderesse vient de découvrir de manière surprenante de nouveaux produits d'addition indole-quinone pouvant être utilisés pour la coloration des fibres kératiniques, en particulier des cheveux humains ou dans des produits de maquillage, et conduisant sans système oxydant à une large gamme de nuances à composante bleue.

Il s'agit de 1,4-naphtoquinones di- ou tri-indolyl-substituées ainsi que leurs dérivés de réduction ou leucodérivés de structure 1,4-naphtalènediol.

La présente invention a donc pour objet ces composés dont la structure est définie ci-après.

Un autre objet de l'invention concerne leurs procédés de préparation.

L'invention concerne également leur utilisation en coloration des matières kératiniques ou comme agent colorant dans des produits de maquillage.

D'autres objets apparaîtront à la lumière de la description et des exemples qui suivent.

Les composés conformes à la présente invention sont caractérisés essentiellement par le fait qu'il s'agit de 1,4-naphtoquinone ou de 1,4-naphtalènediol di- ou tri-indolyl-substitués répondant à l'une des formules (I) ou (II) suivantes :

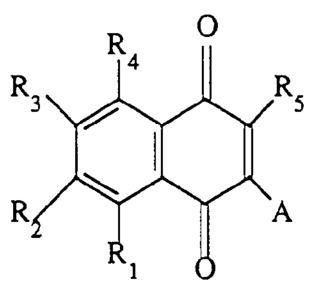

ou

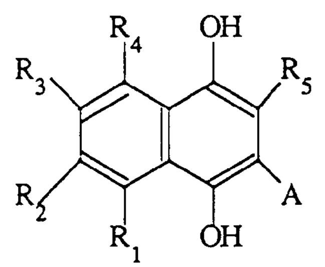

(I) (II)

dans lesquelles :

$R_1$ désigne un atome d'hydrogène, un radical alkyle en $C_1$-$C_4$ ou le groupe OH;

$R_4$ désigne un atome d'hydrogène; un radical alkyle en $C_1$-$C_4$; simultanément avec le radical $R_1$ le groupe OH ou désigne le radical C défini ci-après;

$R_3$ désigne un atome d'hydrogène, un radical alkyle en $C_1$-$C_4$, ou un halogène;

$R_2$ et $R_5$, indépendamment l'un de l'autre, désignent un atome d'hydrogène, un halogène, un radical alkyle en $C_1$-$C_4$, ou un radical B défini ci-après;

un et un seul des radicaux $R_2$ et $R_5$ peut désigner le radical B; $R_1$ désigne OH lorsque $R_4$ désigne le radical C;

A, B et C, indépendamment l'un de l'autre, représentent un groupe de formule (III) :

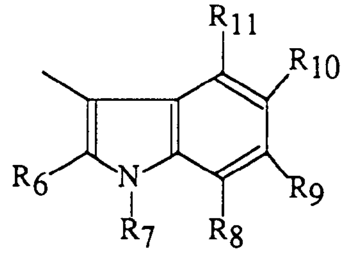

(III)

dans laquelle :

$R_6$ désigne un atome d'hydrogène, un radical alkyle en $C_1$-$C_4$ ou un groupe phényle;

$R_7$ désigne un atome d'hydrogène ou un radical alkyle en $C_1$-$C_4$;

$R_6$, $R_9$, $R_{10}$ et $R_{11}$, indépendamment l'un de l'autre, désignent un atome d'hydrogène, un halogène, un radical alkyle en $C_1$-$C_4$, un groupe benzyloxy, le groupe OH, un radical alcoxy en $C_1$-$C_4$, un groupe $NH_2$ ou amino mono-substitué par un radical alkyle en $C_1$-$C_4$ ou hydroxyalkyle en $C_1$-$C_4$; au plus deux des radicaux $R_6$, $R_9$, $R_{10}$, $R_{11}$ désignent un groupe OH ou alcoxy ou benzyloxy; au plus un des radicaux $R_6$, $R_9$, $R_{10}$ et $R_{11}$ désigne $NH_2$, amino mono-substitué par un alkyle ou un hydroxyalkyle; halogène;

**sous réserve que** :

- $R_4$ représente un groupe C lorsque $R_2$ représente un groupe B et $R_1$ désigne le radical OH;
- $R_6$ désigne un groupe B lorsque $R_4$ ne désigne pas de groupe C; et
- $R_4$ représente un groupe C lorsqu'aucun des radicaux $R_2$ et $R_5$ ne désigne un groupe B et $R_1$ désigne le radical OH.

Les composés préférentiels conformes à la présente invention sont choisis parmi :

(i) ceux de formule :

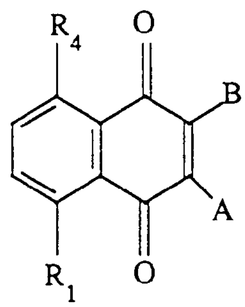

ou

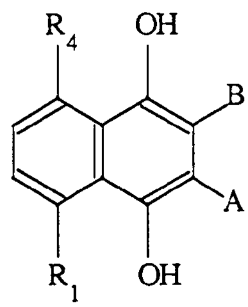

(Ia) (IIa)

dans lesquelles :

$R_1$ et $R_4$ désignent hydrogène ou simultanément OH;

A et B, indépendamment l'un de l'autre, représentent un groupe de formule :

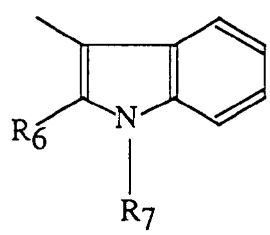

(IIIa)

où $R_6$ et $R_7$ ont les mêmes significations indiquées ci-dessus;

ou (ii) ceux de formule :

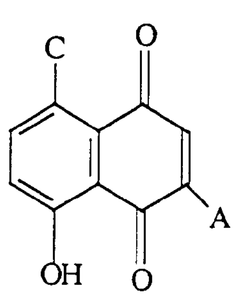

ou

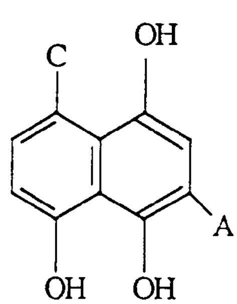

(Ib) (IIb)

où A et C, identiques ou différents, désignent un groupe de formule :

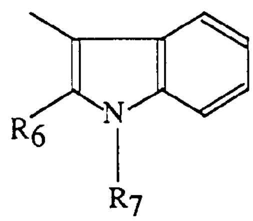

(IIIb)

où $R_6$ a la même signification indiquée ci-dessus,
et $R_7$ représente hydrogène ou alkyle en $C_1$-$C_4$;
ou (iii) ceux de formule :

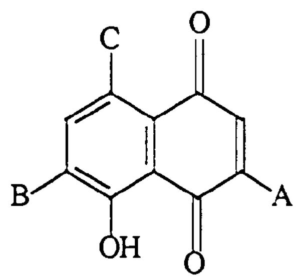

ou

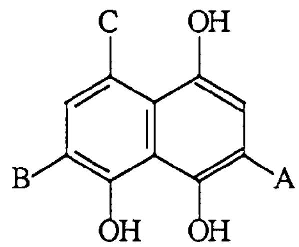

(Ic) (IIc)

où A, B et C, identiques ou différents, désignent un groupe de formule (IIIb) telle que définie ci-dessus.

Les composés plus particulièrement préférés de l'invention sont ceux choisis parmi :

- la 3,8-di(2-phényl 3-indolyl)5-hydroxy 1,4-naphtoquinone
- la 3,8-di(2-méthyl 3-indolyl)5-hydroxy 1,4-naphtoquinone
- la 3-(2-méthyl 3-indolyl)-8(2-phényl 3-indolyl)5-hydroxy-1,4-naphtoquinone
- la 3-(2-phényl 3-indolyl)-8(2-méthyl 3-indolyl)5-hydroxy 1,4-naphtoquinone
- la 3,6,8-tri(2-phényl 3-indolyl)5-hydroxy 1,4-naphtoquinone
- la 2,3-di(2-méthyl 3-indolyl)1,4-naphtoquinone
- la 2,3-di(1,2-diméthyl 3-indolyl)1,4-naphtoquinone
- la 2,3-di(2-phényl 3-indolyl)1,4-naphtoquinone
- la 2-(2-phényl 3-indolyl)3-(2-méthyl 3-indolyl)1,4-naphtoquinone
- la 2-(2-phényl 3-indolyl)3-(1,2-diméthyl 3-indolyl)1,4-naphtoquinone,

ainsi que leurs dérivés de réduction 1,4-naphtalènediol correspondants.

Les composés de l'invention de formule (I) tels que définis précédemment où les substituants A, B et C sont identiques, peuvent être obtenus par réaction de condensation, préférentiellement en milieu acide, de deux ou trois moles d'un même composé indolique de formule (1) :

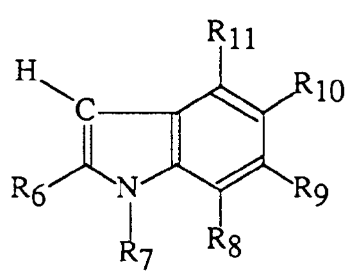

(1)

dans laquelle $R_6$ à $R_{11}$ ont les mêmes significations indiquées dans la formule (III) précédente, par mole d'une 1,4-naphtoquinone de formule (2) :

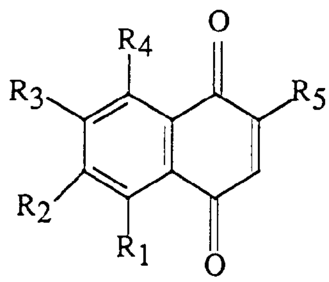

(2)

dans laquelle :

$R_1$ désigne un atome d'hydrogène, un radical alkyle en $C_1$-$C_4$, ou le groupe OH;

$R_4$ désigne un atome d'hydrogène, un radical alkyle en $C_1$-$C_4$ ou simultanément avec $R_1$ le groupe OH;

$R_2$, $R_3$ et $R_5$, indépendamment l'un de l'autre, désignent un atome d'hydrogène, un radical alkyle en $C_1$-$C_4$, un halogène; sous réserve que $R_1$ désigne OH lorsque $R_4$ désigne un hydrogène ou lorsque $R_4$ et $R_2$ désignent un hydrogène.

Les composés de l'invention de formule (I) telle que définie précédemment, dans laquelle les groupes A, B et C peuvent être différents, sont obtenus par 2 ou 3 condensations successives d'unités indoliques de formule (1) telle que définie ci-dessus, sur une 1,4-naphtoquinone de formule (2) telle que définie ci-dessus; les indolyl 1,4-naphtoquinones intermédiaires peuvent être isolées ou non.

On opère de manière préférentielle en catalyse acide en utilisant de l'acide acétique glacial ou un mélange d'acide acétique et de chloroforme.

Les composés particuliers de la présente invention, de formule (Id) :

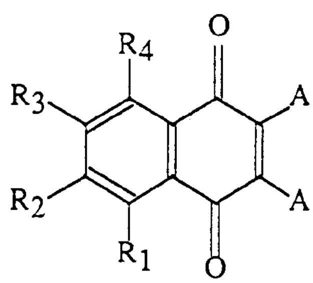

(Id)

dans laquelle $R_1$, $R_2$, $R_3$, $R_4$ et A ont les mêmes significations indiquées dans la formule (I) définie ci-dessus, peuvent être obtenus par réaction de substitution d'un excès de composé indolique de formule (1) telle que définie ci-dessus sur une 1,4-naphtoquinone de formule suivante :

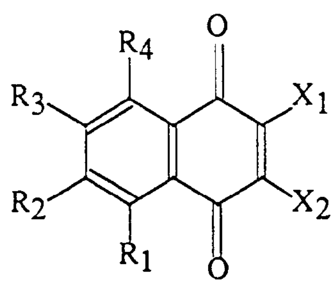

(3)

dans laquelle :

$R_1$ à $R_4$ ont les mêmes significations indiquées dans la formule (Id) ci-dessus;

$X_1$ désigne hydrogène, OH, Cl, Br, $OCH_3$, pipéridyle, thiophényle;

$X_2$ désigne hydrogène, Br ou $OCH_3$;

sous réserve que $X_1$ et $X_2$ ne désignent pas simultanément l'hydrogène.

On opère de manière préférentielle en catalyse acide en utilisant de l'acide acétique glacial à 70°C ou du chloroforme à reflux.

Les composés de l'invention de formule (II) telle que définie ci-dessus, sont obtenus à partir des composés de formule (I) par réduction avec le dithionite de sodium dans une solution d'éther éthylique sous argon.

Le composé de formule (I) est mis en solution dans l'éther éthylique sous argon. La solution obtenue est

agitée en présence de dithionite de sodium en solution aqueuse à température ambiante jusqu'à décoloration totale. On décante, sèche la phase organique et on évapore le solvant.

L'invention concerne également des compositions de coloration des matières kératiniques, de préférence humaines, contenant dans un milieu approprié pour la coloration, au moins un colorant de formule (I) ou (II) telle que définie ci-dessus.

Le milieu approprié pour la coloration est constitué par de l'eau, des solvants ou par un mélange d'eau et d'un ou plusieurs solvant(s) cosmétiquement acceptable(s).

Les solvants sont choisis plus particulièrement parmi les alcools inférieurs en $C_1$-$C_6$, les alkylèneglycols comme l'éthylèneglycol, le propylèneglycol, les éthers de glycol comme les éthers monométhylique, monoéthylique ou monobutylique de l'éthylèneglycol, l'acétate du monoéthyléther de l'éthylèneglycol, les monométhyléthers du propylèneglycol et du dipropylèneglycol, le lactate de méthyle.

Les solvants particulièrement préférés sont l'alcool éthylique et le propylèneglycol.

Les colorants de formule (I) ou (II) sont présents à des concentrations comprises de préférence entre 0,01 et 10% en poids et en particulier entre 0,05 et 5% en poids par rapport au poids total de la composition.

Les compositions conformes à l'invention peuvent également contenir d'autres colorants directs différents de ceux des formules (I) et (II), et notamment des colorants azoïques, anthraquinoniques et les dérivés nitrés benzéniques, et d'autres adjuvants habituellement utilisés en cosmétique tels que des agents tensio-actifs anioniques, cationiques, non-ioniques, amphotères ou leurs mélanges, des épaississants, des parfums, des séquestrants, des agents filmogènes, des agents de traitement des fibres kératiniques, des agents dispersants, des agents de conditionnement des fibres kératiniques, des conservateurs, des agents opacifiants, des pigments, des agents de gonflement des fibres kératiniques.

Les compositions selon l'invention peuvent se présenter sous des formes diverses, telles que des dispersions, des lotions plus ou moins épaissies ou gélifiées, des crèmes, des mousses, des émulsions, des sticks, des poudres. Elles peuvent être conditionnées dans des dispositifs aérosols.

Un autre objet de l'invention concerne un procédé de coloration directe des fibres kératiniques, en particulier des cheveux humains, consistant à appliquer sur ces derniers une composition telle que définie précédemment, l'application étant éventuellement suivie d'un rinçage. Dans le cas où la composition est rincée, le temps de pose varie entre 5 et 45 minutes et de préférence entre 10 et 30 minutes.

Les exemples qui suivent servent à illustrer l'invention sans toutefois présenter un caractère limitatif.

## EXEMPLES DE PREPARATION

### EXEMPLE 1

Synthèse du 3,8-di(2-méthyl 3-indolyl) 5-hydroxy 1,4-naphtoquinone.

**a)** Par condensation indole/juglone : Dans un ballon surmonté d'une arrivée d'argon, on place 10,0 g (57,5 mmoles) de 5-hydroxy 1,4-naphtoquinone recristallisée et 9,05 g (69 mmoles) de 2-méthylindole commercial en solution dans 150 ml d'acide acétique glacial. On agite magnétiquement le mélange à température ambiante pendant 90 minutes au bout desquelles il n'y a plus de quinone. Puis on concentre à l'évaporateur rotatif à 25 ml le mélange réactionnel qui est ensuite coulé sur 1 kg de glace. On agite 15 minutes et on filtre le précipité noir formé et lave par 250 ml d'eau en trois fois. Le solide obtenu est séché et chromatographié sur colonne de gel de silice (40-63 µm) éluée par un mélange chloroforme/cyclohexane. On obtient 2,4 g (7,92 mmoles, Rdt. 28%) de 3-(2-méthyl 3-indolyl) 5-hydroxy 1,4-naphtoquinone et 1,55 g (3,59 mmoles) de 3,8-di(2-méthyl 3-indolyl) 5-hydroxy 1,4-naphtoquinone. Rdt. 19%, PF. 280°C (décomposition).

**b)** Par condensation indole/indolyljuglone : Dans un ballon sont mis en solution 1,0 g (3,30 mmoles) de 3-(2-méthyl 3-indolyl)5-hydroxy 1,4-naphtoquinone obtenue prédécemment et 0,43 g (3,30 mmoles) de 2-méthylindole dans 20 ml d'acide acétique glacial. Les réactifs sont agités à température ambiante pendant 72 heures, puis on concentre sous vide le mélange pour obtenir un résidu noir pâteux qui est repris par 50 ml de dichlorométhane. On lave la solution à l'eau bicarbonatée (5%), on sèche sur sulfate de magnésium calciné sec, et on évapore le solvant. Le résidu cristallin obtenu est chromatographié sur colonne de gel de silice (40-63 µm) éluée par un mélange dichlorométhane/cyclohexane. On isole 0,450 g (1,04 mmole) de 3,8-di(2-méthyl 3-indolyl) 5-hydroxy 1,4-naphtoquinone. Rdt. 63%, PF. 275°C (décomposition).

EXEMPLE 2

Synthèse du 3,8-di(2-phényl 3-indolyl)5-hydroxy 1,4-naphtoquinone.

**a)** Par condensation indole/juglone : On opère comme dans l'exemple 1. Ainsi, 2,0 g (11,5 mmoles) de 5-hydroxy 1,4-naphtoquinone en solution dans 60 ml d'acide acétique glacial sont agités en présence de 4,44 g (22,9 mmoles) de 2-phénylindole à 60°C pendant 192 heures. Après traitement et purification, on obtient 2,53 g (4,55 mmoles) de 3,8-di(2-phényl 3-indolyl)5-hydroxy 1,4-naphtoquinone. Rdt. 79%.

**b)** Par condensation indole/indolyljuglone : Dans un ballon bicol surmonté d'un thermomètre et d'un réfrigérant droit, on dissout à température ambiante 1,0 g (2,74 mmoles) de 3-(2-phényl 3-indolyl)5-hydroxy 1,4-naphtoquinone obtenue précédemment et 1,06 g (5,48 mmoles) de 2-phénylindole dans 15 ml d'acide acétique et 5 ml de chloroforme. Puis on porte la température à 40°C et on agite le mélange pendant 96 heures. Puis on évapore sous vide les solvants et chromatographie directement le résidu sur colonne de gel de silice. On obtient 0,35 g (0,63 mmole) de 3,8-di(2-phényl 3-indolyl)5-hydroxy 1,4-naphtoquinone. Rdt. 23%, PF. 120°C (décomposition).

EXEMPLE 3

Synthèse du 3-(2-méthyl 3-indolyl)8-(2-phényl 3-indolyl)5-hydroxy 1,4-naphtoquinone.

On dissout dans un ballon 500 mg (1,65 mmole) de 3-(2-méthyl 3-indolyl)5-hydroxy 1,4-naphtoquinone et 0,32 g (1,65 mmole) de 2-phénylindole dans 10 ml d'acide acétique glacial. Après 192 heures d'agitation à température ambiante, on évapore le solvant sous vide, reprend le résidu par un minimum de chloroforme et chromatographie sur colonne de gel de silice éluée au chloroforme. On obtient 0,35 g (0,71 mmole) de 3-(2-méthyl 3-indolyl)8-(2-phényl 3-indolyl) 5-hydroxy 1,4-naphtoquinone. Rdt. 86%, PF. 140°C (décomposition).

EXEMPLE 4

Synthèse du 3-(2-phényl 3-indolyl)8-(2-méthyl 3-indolyl) 5-hydroxy 1,4-naphtoquinone.

On opère comme ci-dessus. Ainsi, 1,0 g (2,74 mmoles) de 3-(2-phényl 3-indolyl) 5-hydroxy 1,4-naphtoquinone sont agités dans 15 ml d'acide acétique en présence de 0,72 g (5,48 mmoles) de 2-méthyl indole à température ambiante pendant 192 heures. On isole par chromatographie 0,94 g (1,90 mmole) de 3-(2-phényl 3-indolyl) 8-(2-méthyl 3-indolyl) 5-hydroxy 1,4-naphtoquinone. Rdt. 72%, PF. 101°C (décomposition).

EXEMPLE 5

Synthèse du 3,6,8-tri(2-phényl 3-indolyl) 5-hydroxy 1,4-naphtoquinone.

Dans un ballon bicol surmonté d'un thermomètre et d'un réfrigérant droit, on dissout à température ambiante 1,0 g (2,74 mmoles) de 3-(2-phényl 3-indolyl) 5-hydroxy 1,4-naphtoquinone obtenue précédemment et 1,06 g (5,48 mmoles) de 2-phénylindole dans 15 ml d'acide acétique et 5 ml de chloroforme. Puis on porte la température à 40°C et on agite le mélange pendant 96 heures. Puis on évapore sous vide les solvants et chromatographie directement le résidu sur colonne de gel de silice. On isole 1,00 g (1,34 mmole) de 3,6,8-tri(2-phényl 3-indolyl) 5-hydroxy 1,4-naphtoquinone. Rdt. 49%, PF. 265°C (décomposition).

EXEMPLE 6

Synthèse du 2,3-di(2-méthyl 3-indolyl) 1,4-naphtoquinone.

**a)** Par condensation indole/naphtoquinone : Dans un ballon surmonté d'un réfrigérant, on place 2,0 g (12,7 mmoles) de 1,4-naphtoquinone en solution dans 50 ml d'acide acétique glacial. On ajoute 3,32 g (25,3 mmoles) de 2-méthylindole solide au mélange et on agite à 50°C pendant 192 heures. On coule la solution sur 500 ml d'eau froide et on filtre un précipité noir qui est lavé à l'eau jusqu'à pH neutre et essoré. Ce résidu est solubilisé par 400 ml de dichlorométhane et séché sur sulfate de calcium sec. Puis on concentre la solution à 25 ml et chromatographie tel quel sur colonne de gel de silice éluée au dichlorométhane. On obtient 1,18 g (2,84 mmoles) de 2,3-di(2-méthyl 3-indolyl) 1,4-naphtoquinone. Rdt 45%, PF. 298°C (décompo-sition).

**b)** Par condensation indole/indolylnaphtoquinone : Dans un ballon, on dissout 500 mg (1,74 mmole) de 2-(2-méthyl 3-indolyl)1,4-naphtoquinone et 700 mg (5,34 mmoles) de 2-méthylindole dans 10 ml d'acide acétique. Après 72 heures d'agitation à 70°C et la disparition de l'indolylnaphtoquinone, on évapore le solvant et chromatographie le résidu pour obtenir 450 mg (1,08 mmole) de 2,3-di(2-méthyl 3-indolyl) 1,4-naphtoquinone. Rdt. 62%, PF. 270°C (décomposition).

EXEMPLE 7

Synthèse du 2,3-di(1,2-diméthyl 3-indolyl) 1,4-naphtoquinone.

**a)** Par condensation indole/naphtoquinone : On procède comme ci-dessus pour l'exemple 6. Ainsi, 1,0 g (6,33 mmoles) de 1,4-naphtoquinone est agitée à 50°C pendant 192 heures dans l'acide acétique en présence de 4,16 g (28,5 mmoles) de 1,2-diméthylindole. On obtient 0,915 g (2,06 mmoles) de 2,3-di(1,2-diméthyl 3-indolyl) 1,4-naphtoquinone. Rdt. 65%, PF. 293°C.
**b)** Par condensation indole/indolylnaphtoquinone : On opère comme ci-dessus pour l'exemple 6. Ainsi, 1,00 g (3,32 mmoles) de 2-(1,2-diméthyl 3-indolyl) 1,4-naphtoquinone et 1,46 g (10 mmoles) de 1,2-diméthylindole donnent, après 168 heures d'agitation à 50°C, 1,30 g (2,93 mmoles) de 2,3-di(1,2-diméthyl 3-indolyl) 1,4-naphtoquinone. Rdt. 88%, PF. 292°C.

EXEMPLE 8

Synthèse du 2,3-di(2-phényl 3-indolyl) 1,4-naphtoquinone.

**a)** Par condensation indole/naphtoquinone : On opère comme ci-dessus pour l'exemple 6. On agite 15,8 g (100 mmoles) de 1,4-naphtoquinone avec 19,3 g (100 mmoles) de 2-phénylindole dans l'acide acétique à 65°C pendant 192 heures et on obtient 4,10 g (7,48 mmoles) de 2,3-di(2-phényl 3-indolyl) 1,4-naphtoquinone. Rdt. 15%, PF. > 300°C.
**b)** Par condensation indole/indolylnaphtoquinone : On suit le mode opératoire de l'exemple 6. Dans 30 ml d'acide acétique, on dissout 1,0 g (2,86 mmoles) de 2-(2-phényl 3-indolyl) 1,4-naphtoquinone et 2,77 g (14,3 mmoles) de 2-phénylindole et on agite à 70°C pendant 840 heures. Après chromatographie, on isole 1,10 g (2,04 mmoles) de 2,3-di(2-phényl 3-indolyl) 1,4-naphtoquinone. Rdt. 71%. PF. > 300°C.

EXEMPLE 9

Synthèse du 2-(2-phényl 3-indolyl) 3-(2-méthyl 3-indolyl) 1,4-naphtoquinone.

**a)** A partir d'indolylnaphtoquinone : On opère comme ci-dessus pour l'exemple 6. On agite 1,00 g (3,48 mmoles) de 2-(2-méthyl 3-indolyl) 1,4-naphtoquinone et 1,35 g (6,98 mmoles) de 2-phénylindole dans 20 ml d'acide acétique glacial à température ambiante pendant 240 heures. On isole, après chromatographie, 0,35 g (0,73 mmoles) de 2-(2-phényl 3-indolyl) 3-(2-méthyl 3-indolyl) 1,4-naphtoquinone. Rdt. 21% (décomposition à 90°C).
**b)** A partir d'indolylnaphtoquinone : En procédant comme ci-dessus pour l'exemple 6, 2,00 g (5,73 mmoles) de 2-(2-phényl 3-indolyl) 1,4-naphtoquinone et 1,50 g (11,46 mmoles) de 2-méthylindole conduisent, après 24 heures d'agitation dans l'acide acétique à température ambiante, à 2,44 g (5,10 mmoles) de 2-(2-phényl 3-indolyl) 3-(2-méthyl 3-indolyl) 1,4-naphtoquinone. Rdt. 89% (décomposition à 95°C).

EXEMPLE 10

Synthèse du 2-(2-phényl 3-indolyl) 3-(1,2-diméthyl 3-indolyl) 1,4-naphtoquinone.

**a)** A partir d'indolylnaphtoquinone : Selon le mode opératoire décrit à l'exemple 6, 0,50 g (1,43 mmole) de 2-(2-phényl 3-indolyl) 1,4-naphtoquinone et 0,42 g (2,87 mmoles) de 1,2-diméthylindole réagissent dans 10 ml d'acide acétique à 70°C pendant 15 heures pour donner 0,70 g (1,42 mmole) de 2-(2-phényl 3-indolyl) 3-(1,2-diméthyl 3-indolyl) 1,4-naphtoquinone. Rdt. 99%, PF. 204°C.
**b)** A partir d'indolylnaphtoquinone : Comme précédemment, 1,00 g (3,32 mmoles) de 2-(2-phényl 3-indolyl) 1,4-naphtoquinone et 1,93 g (10 mmoles) de 2-phénylindole, agités dans 10 ml d'acide acétique à 50°C pendant 240 heures, conduisent, après chromatographie, à 15 mg (0,03 mmole) de 2-(2-phényl 3-indolyl) 3-(1,2-diméthyl 3-indolyl) 1,4-naphtoquinone. PF. 212° C.

EXEMPLE 11

Synthèse de la 2,3-di(2-méthyl 3-indolyl) 1,4-naphtoquinone par réaction de substitution à partir de :

**a)** La 2-hydroxy 1,4-naphtoquinone : En utilisant le chloroforme à reflux en catalyse acide, l'action de 11,3 g (86,1 mmoles) de 2-méthyl indole sur 5,0 g (28,7 mmoles) de 2-hydroxy 1,4-naphtoquinone pendant 6 heures au reflux, conduit à 2,30 g (5,53 mmoles) de la 2,3-di(2-méthyl 3-indolyl) 1,4-naphtoquinone. PF. 298°C.

**b)** La 2-méthoxy 1,4-naphtoquinone : En opérant comme en **a**), 1,00 g (5,31 mmoles) de 2-méthoxy 1,4-naphtoquinone et 2,10 g (16 mmoles) de 2-méthylindole donnent, après 72 heures, 104 mg (0,25 mmole) de 2,3-di(2-méthyl 3-indolyl) 1,4-naphtoquinone. PF. 290°C.

**c)** La 2-chloro 1,4-naphtoquinone : Selon le mode opératoire de **a**), 1,00 g (5,2 mmoles) de 2-chloro 1,4-naphtoquinone réagissent avec 2,05 g (15,6 mmoles) de 2-méthylindole pendant 72 heures pour donner 0,33 g (0,79 mmole) de 2,3-di(2-méthyl 3-indolyl) 1,4-naphtoquinone. PF. 292°C.

**d)** La 2-(1-pipéridyl) 1,4-naphtoquinone : En opérant selon les conditions du mode opératoire défini en **a**), 1,50 g (6,22 mmoles) de 2-(1-pipéridyl) 1,4-naphtoquinone et 2,6 g (19,8 mmoles) de 2-méthyl indole conduisent, après 168 heures d'agitation à 70°C, à 20 mg (0,05 mmole) de 2,3-di(2-méthyl 3-indolyl) 1,4-naphtoquinone. PF. 295°C.

**e)** La 2-thiophényl 1,4-naphtoquinone : En opérant en catalyse avec l'acide acétique glacial à 70°C, 1,50 g (5,64 mmoles) de 2-thiophényl 1,4-naphtoquinone et 1,11 g (8,46 mmoles) de 2-méthylindole conduisent, après 240 heures d'agitation à 25 mg (0,06 mmole) de 2,3-di(2-méthyl 3-indolyl) 1,4-naphtoquinone. PF. 295°C.

**f)** La 2-bromo 3-méthoxy 1,4-naphtoquinone : Dans un ballon purgé à l'argon, on agite 13,30 g (50 mmoles) de 2-bromo 3-méthoxy 1,4-naphtoquinone et 6,56 g (50 mmoles) de 2-méthylindole dans 250 ml d'éthanol absolu. On porte au reflux pendant 72 heures, puis on évapore le solvant. Le résidu obtenu est chromatographié sur colonne de gel de silice. On obtient ainsi 14,27 g (40 mmoles) de 2-bromo 3-(2-méthyl 3-indolyl) 1,4-naphtoquinone. Rdt. 78% et 1,03 g (2,48 mmoles) de 2,3-di(2-méthyl 3-indolyl) 1,4-naphtoquinone. PF. 293°C.

**g)** La 2,3-dibromo 1,4-naphtoquinone : En procédant comme ci-dessus, 15,80 g (50 mmoles) de 2,3-dibromo 1,4-naphtoquinone réagissent sur 6,56 g (50 mmoles) de 2-méthylindole, pour fournir 11,34 g (31 mmoles) de 2-bromo 3-(2-méthyl 3-indolyl) 1,4-naphtoquinone. Rdt. 62%, et 2,10 g (5,05 mmoles) de 2,3-di(2-méthyl 3-indolyl) 1,4-naphtoquinone. PF. 298°C.

**TABLEAU 1**

| Valeurs d'absorption U.V. - Visible des 3,8-diindolyl 5-hydroxy 1,4-naphtoquinones en solution dans le THF 99,8%. | |
|---|---|
| Exemples | λ max (nm) ($log_{10}$ ε) |
| 2 | 224(4,75) ; 282(4,33) ; 416(3,48) ; 528(3,49) |
| 1 | 201(4,26) ; 220(4,60) ; 236(4,54) ; 298(4,40) ; 427(3,50) ; 532(3,48) |
| 3 | 212(5,25) ; 288(5,05) ; 428(4,20) ; 533(4,23) |
| 4 | 230(4,58) ; 282(4,37) ; 428(3,49) ; 540(3,46) |
| 6 | 209(4,67) ; 219(4,67) ; 283(4,33) ; 290(4,32) 550(3,36) |
| 7 | 210(5,05) ; 228(5,30) ; 282(4,94) ; 291(4,94) 548(3,91) |
| 8 | 201(4,86) ; 211(5,04) ; 237(4,90) ; 295(4,77) 516(3,63) |
| 9 | 205(4,72) ; 215(4,67) ; 291(4,37) ; 571(3,42) |
| 10 | 201(4,64) ; 211(4,87) ; 227(4,77) ; 293(4,48) 531(3,60) |

Dans tous les exemples 1 à 11 de préparation, les spectres RMN $^{13}C$ et $^{1}H$ sont conformes à la structure attendue.

ε = coefficient d'extinction molaire.

## EXEMPLES DE COMPOSITIONS

### EXEMPLE A

On prépare une lotion colorante ayant la composition suivante :

- 2,3 di(2-méthyl 3-indolyl)1,4-naphtoquinone 0,5 g
- Copolymère acétate de vinyle/acide crotonique/polyéthylèneglycol, vendu sous la dénomination ARISTOFLEX A par la Société HOESCHT 1,5 g
- Alcool éthylique absolu 40 g
- Triéthanolamine qs pH=7
- Eau déminéralisée qsp 100 g

Cette lotion de mise en plis est appliquée sur des cheveux blancs à 100%. Elle leur confère une coloration violette.

### EXEMPLE B

- 2(2-phényl 3-indolyl)3(2-méthyl 3-indolyl) 1,4-naphtoquinone 0,2 g
- Copolymère acétate de vinyle/acide crotonique/polyéthylèneglycol, vendu sous la dénomination ARISTOFLEX A par la Société HOESCHT 1,5 g
- Alcool éthylique absolu 40 g
- Triéthanolamine qs pH=7
- Eau déminéralisée qsp 100 g

On applique cette lotion de mise en plis sur des cheveux blancs à 100%. Après séchage, les cheveux sont colorés en rose, légèrement bleuté.

### EXEMPLE C

On prépare la composition tinctoriale suivante :

- 2,3 di(2-méthyl 3-indolyl)1,4-naphtoquinone 0,42 g
- Lauryléthersulfate de sodium à 2 moles d'oxyde d'éthylène en solution aqueuse à 28% 20 g
- Alcool éthylique 35 g
- Monobutyléther d'éthylèneglycol 5 g
- Acide acétique qs pH=5
- Eau déminéralisée qsp 100 g

Cette composition est appliquée pendant 20 minutes sur des cheveux gris à 90% de blancs. Après rinçage et séchage, les cheveux sont colorés dans une nuance violine à reflets cendrés.

### EXEMPLE D

On prépare une lotion colorante ayant la composition suivante :

- 3,8-di-(2-méthyl 3-indolyl)5-hydroxy 1,4-naphtoquinone 0,3 g
- Polyvinylpyrrolidone vendue sous la dénomination PVP K30 par la Société GAF 2 g
- 2-butoxyéthanol 10 g
- Alcool éthylique 50 g
- Eau qsp 100 g

Le pH est égal à 6,6

On applique cette lotion de mise en plis sur des cheveux décolorés. Après séchage, les cheveux sont colorés en châtain clair.

## Revendications

**1.** Composés répondant à l'une des deux formules suivantes :

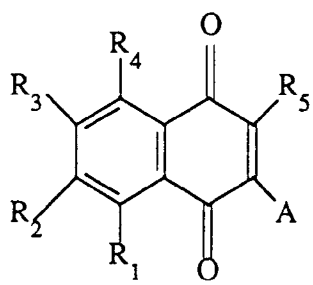

ou

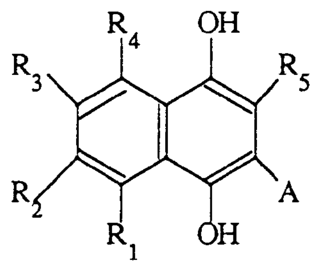

(I) (II)

dans lesquelles :

$R_1$ désigne un atome d'hydrogène, un radical alkyle en $C_1$-$C_4$ ou le groupe OH;

$R_4$ désigne un atome d'hydrogène; un radical alkyle en $C_1$-$C_4$; simultanément avec le radical $R_1$, le groupe OH ou un radical C;

$R_2$ et $R_5$, identiques ou différents, désignent un atome d'hydrogène, un halogène, un radical alkyle en $C_1$-$C_4$ ou un radical B; un et un seul pouvant désigner B;

$R_3$ désigne un atome d'hydrogène, un radical alkyle en $C_1$-$C_4$ ou un halogène;

A, B et C, identiques ou différents, désignent un groupe de formule (III) :

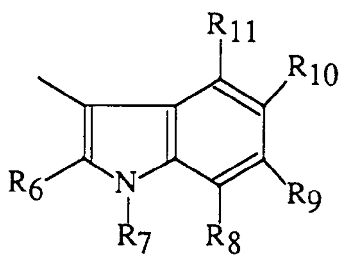

(III)

dans laquelle :

$R_6$ désigne un atome d'hydrogène, un radical alkyle en $C_1$-$C_4$ ou un groupe phényle;

$R_7$ désigne un atome d'hydrogène ou un radical alkyle en $C_1$-$C_4$;

$R_8$, $R_9$, $R_{10}$ et $R_{11}$, identiques ou différents, désignent un atome d'hydrogène, un halogène, un radical alkyle en $C_1$-$C_4$, un groupe benzyloxy, le groupe OH, un radical alcoxy en $C_1$-$C_4$, un groupe $NH_2$ ou amino mono-substitué par un radical alkyle en $C_1$-$C_4$ ou hydroxyalkyle en $C_1$-$C_4$; au plus deux des radicaux $R_8$, $R_9$, $R_{10}$, $R_{11}$ désignent un groupe OH, alcoxy ou benzyloxy; au plus un des radicaux $R_8$, $R_9$, $R_{10}$ et $R_{11}$ désigne $NH_2$, amino mono-substitué par un alkyle ou par un hydroxyalkyle ou halogène;

sous réserve que :

- $R_1$ désigne OH lorsque $R_4$ représente un radical C;
- $R_4$ représente un groupe C lorsque $R_2$ représente un groupe B ;
- $R_8$ désigne un groupe B lorsque $R_4$ ne désigne pas de groupe C;
- $R_4$ représente un groupe C lorsqu'aucun des radicaux $R_2$ et $R_8$ ne désigne de groupe B.

**2.** Composés selon la revendication 1, caractérisés par le fait qu'ils sont choisis parmi :

- la 3,8-di(2-phényl 3-indolyl)5-hydroxy 1,4-naphtoquinone
- la 3,8-di(2-méthyl 3-indolyl)5-hydroxy 1,4-naphtoquinone
- la 3-(2-méthyl 3-indolyl)-8(2-phényl 3-indolyl)5-hydroxy-1,4-naphtoquinone
- la 3-(2-phényl 3-indolyl)-8(2-méthyl 3-indolyl)5-hydroxy 1,4-naphtoquinone
- la 3,6,8-tri(2-phényl 3-indolyl)5-hydroxy 1,4-naphtoquinone
- la 2,3-di(2-méthyl 3-indolyl)1,4-naphtoquinone
- la 2,3-di(1,2-diméthyl 3-indolyl)1,4-naphtoquinone
- la 2,3-di(2-phényl 3-indolyl)1,4-naphtoquinone
- la 2-(2-phényl 3-indolyl)3-(2-méthyl 3-indolyl)1,4-naphtoquinone
- la 2-(2-phényl 3-indolyl)3-(1,2-diméthyl 3-indolyl)1,4-naphtoquinone,

ainsi que leurs dérivés de réduction 1,4-naphthalènediol correspondants.

**3.** Procédé de préparation des composés de formule (I) tels que définis dans la revendication 1, caractérisé par le fait que l'on effectue une condensation en catalyse acide d'un excès d'une unité indolique ou des condensations successives en catalyse acide d'unités indoliques de formule (1) :

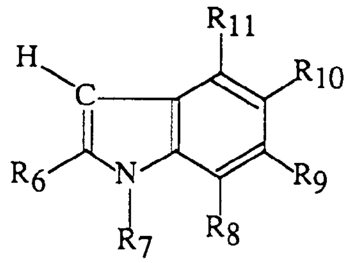

(1)

dans laquelle $R_6$ à $R_{11}$ ont les mêmes significations indiquées dans la revendication 1, sur une 1,4-naphtoquinone de formule (2) :

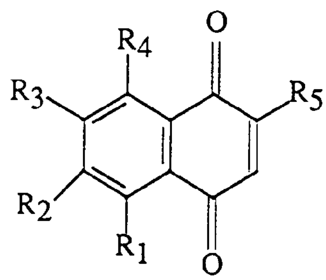

(2)

dans laquelle :

$R_1$ désigne hydrogène, alkyle en $C_1$-$C_4$, OH;

$R_2$, $R_3$, $R_6$, identiques ou différents, désignent hydrogène, halogène ou alkyle en $C_1$-$C_4$;

$R_4$ désigne hydrogène, alkyle en $C_1$-$C_4$ ou simultanément avec $R_1$ le groupe OH, sous réserve que $R_1$ désigne OH lorsque $R_4$ désigne hydrogène ou lorsque $R_2$ et $R_4$ désignent hydrogène.

**4.** Procédé de préparation des composés de formule (Id) :

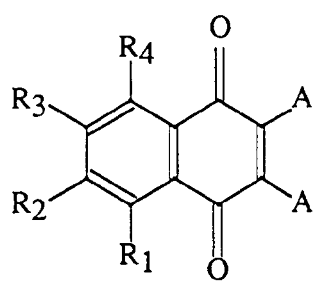

(Id)

dans laquelle $R_1$, $R_2$, $R_3$, $R_4$ et A ont les mêmes significations indiquées dans la formule (I) de la revendication 1, caractérisé par le fait que l'on effectue une réaction de substitution en catalyse acide, d'un excès de composé indolique de formule (1) :

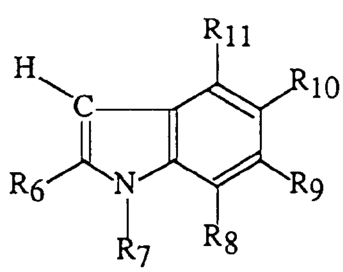

(1)

dans laquelle $R_6$ à $R_{11}$ ont les mêmes significations indiquées dans la revendication 3 sur une 1,4-naphtoquinone de formule (3) :

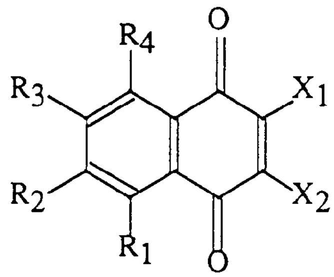

(3)

dans laquelle :

les radicaux $R_1$ à $R_4$ ont les mêmes significations indiquées ci-dessus;

$X_1$ désigne hydrogène, OH, Cl, Br, $OCH_3$, pipéridyle, thiophényle;

$X_2$ désigne hydrogène, Br ou $OCH_3$, sous réserve que $X_1$ et $X_2$ ne désignent pas simultanément hydrogène.

**5.** Procédé de préparation d'un composé de formule (II) telle que définie dans la revendication 1, caractérisé par le fait que l'on effectue une réduction du composé de formule (I), dissout dans une solution d'éther éthylique, sous argon, à l'aide d'une solution aqueuse de dithionite de sodium, à température ambiante.

**6.** Utilisation des composés de formule (I) ou (II) tels que définis dans la revendication 1, pour la coloration des matières kératiniques.

**7.** Composition de coloration des matières kératiniques, en particulier humaines, caractérisée par le fait qu'elle contient au moins dans un milieu approprié pour la coloration, un composé répondant à la formule (I) ou (II) telle que définie dans la revendication 1.

**8.** Composition selon la revendication 7, caractérisée par le fait que le composé de formule (I) ou (II) est présent dans des proportions comprises entre 0,01 et 10% en poids par rapport au poids total de la composition.

**9.** Composition selon la revendication 7 ou 8, caractérisée par le fait que le milieu approprié pour la teinture est constitué par de l'eau, un solvant ou un mélange d'eau et d'un ou plusieurs solvant(s) choisi(s) parmi les alcools inférieurs en $C_1$-$C_6$, les alkylèneglycols, les éthers de glycol comme les éthers monométhylique, monoéthylique ou monobutylique de l'éthylèneglycol, l'acétate de monoéthyléther de l'éthylèneglycol; les monométhyléthers du propylèneglycol et du dipropylèneglycol; le lactate de méthyle.

**10.** Composition selon l'une quelconque des revendications 7 à 9, caractérisée par le fait qu'elle contient en plus des colorants directs autres que ceux de formule (I) ou (II), des agents tensio-actifs anioniques, cationiques, non-ioniques, amphotères ou leurs mélanges, des épaississants, des parfums, des séquestrants, des agents filmogènes, des agents de traitement des fibres kératiniques, des dispersants, des opacifiants, des pigments, des agents de conditionnement des fibres kératiniques, des conservateurs, des agents de gonflement des fibres kératiniques ou autres adjuvants habituellement utilisés en cosmétique.

**11.** Composition selon l'une quelconque des revendications 7 à 10, caractérisée par le fait qu'elle se présente sous forme de lotion plus ou moins épaissie ou gélifiée, de dispersion, d'émulsion, de crème, de mousse, de stick, de poudre, et qu'elle peut être conditionnée dans un dispositif aérosol.

**12.** Procédé de coloration directe des fibres kératiniques, en particulier des cheveux humains, caractérisé par le fait que l'on applique sur ces derniers au moins une composition telle que définie dans l'une quelconque des revendications 7 à 11, cette application étant éventuellement suivie d'un rinçage.

**13.** Utilisation des composés de formule (I) ou (II) tels que définis dans la revendication 1, comme agent colorant dans des produits de maquillage.

Office européen des brevets

# RAPPORT DE RECHERCHE EUROPEENNE

Numero de la demande

EP 93 40 0626

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,A | EP-A-0 376 776 (L'ORÉAL)<br>* revendications *<br>--- | 1,6 | C07D209/12<br>A61K7/13 |
| D,A | EP-A-0 460 996 (L'ORÉAL)<br>* revendications *<br>----- | 1,6 | |
| | | | **DOMAINES TECHNIQUES RECHERCHES (Int. Cl.5 )** |
| | | | C07D<br>A61K |

Le présent rapport a été établi pour toutes les revendications

| Lieu de la recherche | Date d'achèvement de la recherche | Examinateur |
|---|---|---|
| LA HAYE | 17 JUIN 1993 | VAN BIJLEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)